# EUROPEAN PATENT APPLICATION

(11) **EP 2 275 157 A2**
(43) Date of publication of application: **19.01.2011**
(21) Application number: 10185674.8
(22) Date of filing: 16.05.2003
(51) Int. Cl.: A61M 5/172, A61B 5/02

(54) **Kits of medical supplies for sedation and analgesia**

(30) Priority: 16.05.2002 US 378068 P
(62) Divisional of application: 03753052.4
(71) Applicant: Scott Laboratories, Inc., Lubbock, TX 79415 (US)
(72) Inventor: Hickle, Randall, S., Lubbock, TX 79407 (US); Lampotang, Samsun, Gainesville, FL 32608 (US)
(74) Representative: Leihkauf, Steffen Falk

(57) **Abstract**

The application relates to kits of supplies and components for the computer assisted IV drug infusion administration device where those supplies and components may be disposable or re-usable. In one embodiment of the single-patient use disposable components are utilized with a computer assisted IV drug infusion administration device to prevent potential cross-contamination and drug carry-over from a previous infusion to a different patient.

## Description

### BACKGROUND OF THE INVENTION

### Field of the Invention.

The invention of this application relates generally to automated drug infusion devices. More specifically, the invention relates to kits of supplies and components for the computer assisted IV drug infusion administration device where those supplies and components may be disposable or re-usable.

### Description of Related Art.

Mechanically controlled infusion of a liquid drug from a reservoir directly to a patient is a useful process of administering a drug. An electro-mechanically controlled infusion process often provides a much steadier and more accurate administration of a drug than is possible from a human manually giving injections. By maintaining a steady or accurate flow rate of drug, an electro-mechanically controlled infusion device can ensure that the concentration or amount of drug entering a patient's circulatory system remains steadily within the drug's therapeutic range.

Various medical devices for controlling the infusion of a liquid directly to a patient are known. Certain of these devices utilize pumping mechanisms to deliver liquid drugs from a reservoir such as a syringe, a collapsible bag, or a vial to a patient supply tube. One example of such a device, shown in U.S. Patent No. 6,186,977, includes a liquid drug supply in a collapsible bag and an infusion pump, which draws drug directly from the supply and moves it along a flow passage to a patient supply tube.

Certain of these medical devices further utilize drug pump cassettes, which provide a rigid housing and pressure plate that interact with the pumping mechanisms of the devices. These cassettes serve as intermediary devices between drug containers and patient supply lines.

A typical cassette includes a passage, which is acted upon by the pumping mechanism of an infusion device to move the drug along to the supply line.

One example of a cassette for use with a drug pumping system, shown in U.S. Patent No. 6,165,154, has a fluid passage and a collapsible pressure conduction chamber for generating a pressure gradient to move drug along the passage. Certain other cassettes are known which provide means for moving drug along a flow channel without the drug interacting directly with the pumping mechanism. One example of this other type of cassette, shown in U.S. Patent No. 6,202,708, provides a large chamber for mixing a powdered drug with a liquid solvent. The cassette also includes a pressure plate, which supports a fluid flow passage against which a peristaltic pump may act to move the liquid along to a patient delivery tube.

Certain liquid infusion devices which provide means for removing air that has entered their flow passages are also known. However, the means of these devices require an inefficient purging process which in turn requires human intervention and/or knowledge of the exact dead-space volume of all of the liquid passages in the system in order to flush the trapped air from the passages without losing excessive amounts of the drug.

There are also known drug infusion systems which are provided with computers that can track the volume of the liquid drug remaining in a container by tracking internal encoder counts within the pumping mechanism. A problem with tracking volume based on internal effects, though, is that if there is an inconsistency with respect to a component within the infusion device, the calculated volume of drug infused may be incorrect and yet would nonetheless appear to be consistent with the operation of the device.

There are further drawbacks to the efficiency and safety of all of the aforementioned devices. One such drawback is that the known drug infusion devices do not allow for a cost effective means of disposing of those elements which come in direct contact with the drug. It is beneficial from a quality control and patient safety standpoint to replace those parts of a liquid drug infusion device which directly contact the drug upon the completion of each infusion process. Disposal and replacement provide an efficient means of starting each infusion process with clean components that are free from residual drug remaining from an earlier infusion or from vectors for cross-contamination from the previous patient. Some parts of the aforementioned devices, such as the drug pump cassettes, are large and bulky and so are expensive and clumsy to replace after a single-patient use.

Another drawback of the above devices is that certain of their components, such as the drug containers, cassettes, and flow passages, cannot be replaced during an infusion process, i.e. while the pumping mechanism is active, without introducing air bubbles into the system. Air bubbles may also be introduced into the systems if these components are accidentally removed from the device during an infusion process. Air bubbles that are not removed from the flow passages of a direct-to-patient infusion system can be dangerous to the patient's circulatory system.

Deaths have resulted from erroneous delivery of potent pain killers such as morphine. Thus, a means of controlling the infusion rate of a drug based on a measurement or inference of an effect of the delivered drug on the patient would be beneficial. Such a means of control would be especially desirable during outpatient, ambulatory, gastrointestinal, cardiac catheterization, imaging and other procedures at remote and/or minimally staffed or equipped locations such as, among others, office-based surgery, imaging, dermatology suites and far-forward military medical outposts where anesthesia and sedation and analgesia are provided with the concomitant risk of loss of consciousness and apnea.

A kit generally comprises two or more components bundled or otherwise grouped together as one package. An example of such a kit in a medical context is a first-aid kit having scissors, medical tape and alcohol preps. Disposable kits of medical supplies, such as tracheostomy kits for example, are also available.

Such kits for systems for sedation and analgesia may enhance efficiency by simplifying inventory management as well as improving safety by specifying, organizing, and providing all required components. When a kit is comprised of disposable items, the disposable nature eliminates the need for collection, storage and sterilization of used supplies and the potential for cross-contamination from improperly sterilized supplies. Conversely, re-usable supplies tend to be of higher quality than disposables because they are designed and manufactured to last through repeated use cycles. The re-usable nature helps to amortize the cost of acquisition over multiple uses such that the acquisition cost per use may be lower than of disposables. Depending on labor costs, cost of collection, sterilization and repackaging of used components and the legal liability from improper sterilization of re-usable supplies, re-usable items may also have a lower cost per use. Some of the re-usable supplies may be recycled or reconditioned to yield equipment of higher quality and lower cost-per-use than corresponding disposable equipment.

### BRIEF SUMMARY OF THE INVENTION

The present invention solves the aforementioned drawbacks of and needs from automated drug infusion devices by providing an infusion system with a drug pump cassette that features disposable components, external redundant volume tracking, air removal and automatic purge capabilities, component lockout mechanisms, redundant automated anti free flow devices and automated modulation of infusion rate based on measured or inferred effects on the patient.

It is an object of the present invention to provide a computer assisted IV drug infusion administration device with single-patient use disposable components to prevent potential cross-contamination and drug carry-over from a previous infusion to a different patient. Components of this aspect of the invention that may be disposable may include, among other items, drug containers, infusion tubing, pressure plates, infusion line connectors, anti-reflux valves, EKG pads or skin electrodes, IV catheters, and oxygen delivery, gas sampling and respiratory apparatuses and responsiveness query devices.

It is a further object of the present invention that some of these disposable components are integrated into a single-use cassette for the transmission of drug from the containers to the patient. The cassette is fixed to the administration device with a single-motion snap-on action. The cassette is of a fixed form so that its components align with the permanent components of the device upon the single-motion snap on action. For example, the delivery conduit is positioned at the active portion of a pumping mechanism on the administration device when the cassette is fitted into place.

The present invention allows for the drug vial to be removed and replaced during a given procedure without requiring the user to purge the infusion line of air. A vial-lockout mechanism is provided to prevent removal of the vial while the pump is running. To prevent free flow, various redundant infusion line lockouts automatically close off the drug flow lumen when the cassette is not inserted into the administration device. The lockouts are provided to guard against the pumping mechanism transporting air bubbles to the patient and against the free uncontrolled flow of drug by gravity feed to the patient. To prevent the air bubbles from reaching the patient if the lockout mechanisms fail, an air in line (AIL) detector acts as back-up safety device.

The computer assisted IV drug infusion administration device provides an efficient means of controlling the flow of drug from a drug container such as a vial, syringe or collapsible bag to a manifold connector (containing anti-reflux valves) where the drug may be combined with an IV solution before administration to the patient. Computer control allows accurate flow rates and precise control of those flow rates for infusion and purging procedures as well as automated purging without the need for the user to intervene or remember to purge the line. Flow rate accuracy, combined with the knowledge of the deadspace in the IV infusion set (acquired via a quality assurance module associated with the drug cassette), ensures the conservation of expensive drugs such as propofol, which may be wasted during manual control of the same procedures.

The present invention also provides kits of supplies and components for the computer assisted IV drug infusion administration device where those supplies and components may be disposable or re-usable, The kits may be engineered so as to better provide efficient, safe, and easy use of the supplies and components. The kits and the supplies and components themselves may also be tagged with identifying indicia for quality assurance purposes.

### BRIEF DESCRIPTION OF THE DRAWINGS

FIG. 1A shows a perspective view of one embodiment of the computer assisted IV drug infusion administration device;
FIG. 1B depicts an 0₂ control/respiratory monitoring device;
FIG. 2 shows a data flow diagram of the computer assistance to the infusion process;
FIG. 3A shows a perspective view of one embodiment of the cassette;
FIG. 3B shows a different perspective view of one embodiment of the cassette that highlights how the drug delivery conduit can be removed from the cassette;
FIG. 4 shows a front cross-sectional view of an alternative embodiment of the cassette extension with a drug container in place thereon;
FIG. 5 shows a top cross-sectional view of one embodiment of the cassette fitted with the administration device;
FIG. 6 shows a perspective view of one embodiment of a redundant volume tracking system;
FIG. 7 shows one embodiment of the anti-reflux valve and IV manifold connector;
FIG. 8 shows a block diagram of the liquid and air flow between various components;
FIG. 9 shows a block diagram of the mechanisms for redundant volume tracking;
FIG. 10 shows a block diagram of the mechanisms for the automatic shut off of the pumping mechanism;
FIG. 11 shows a block diagram of the parameters used with the quality assurance modules;

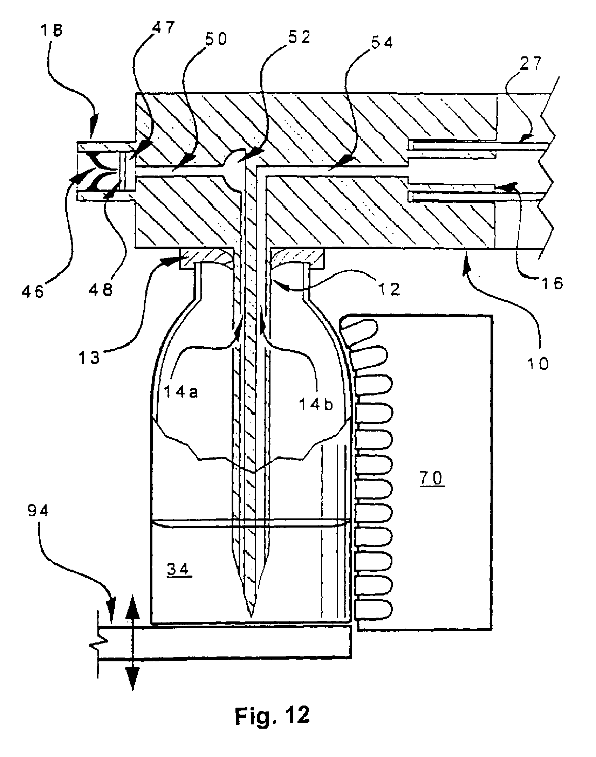

FIG. 12 shows a front cross-sectional view of one embodiment of the cassette extension with a drug container suspended therefrom; and
FIG. 13 shows a kit containing two disposable components associated with the administration device.

### DETAILED DESCRIPTION OF THE PREFERRED EMBODIMENTS

The embodiments described below are not intended to limit the invention to the precise forms disclosed. The embodiments are chosen and described in order to explain the principles of the invention and its applications and uses, and thereby enable others skilled in the art to make and use the invention.

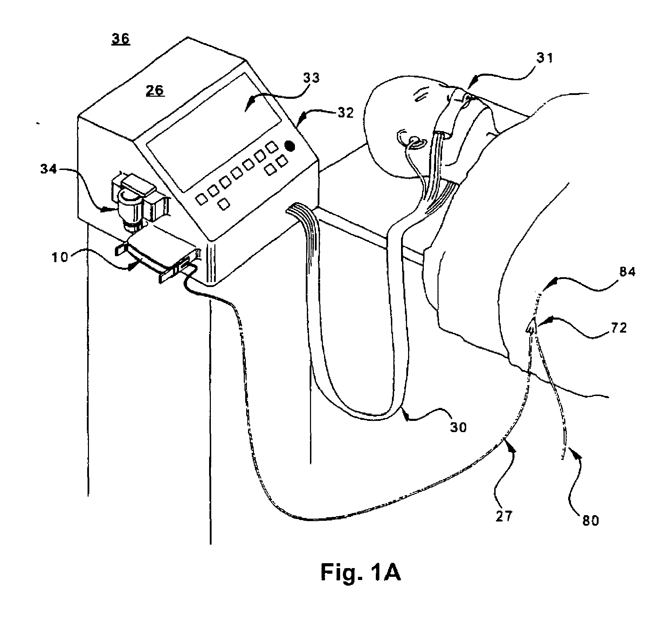

**FIG. 1A** shows an external view of the computer assisted IV drug infusion administration device **36** of the present invention. The system includes a housing **26** for the user interface **32** and pumping mechanism **56,** as well as ports for the attachment or insertion of, drug container **34,** a detachable cassette **10** for receiving the drug container **34** and patient interface devices such as an oro-nasal device **31.** Drug flows from the cassette to the patient via the intravenous infusion line or drug flow conduit **27.** Intravenous fluid, if used, flows to the patient via a separate infusion line **80.** Lines **80** and **27** merge at connector **72.** Fluid flows from the connector **72** to the patient via the IV catheter **84** that is inserted in a vein of the patient.

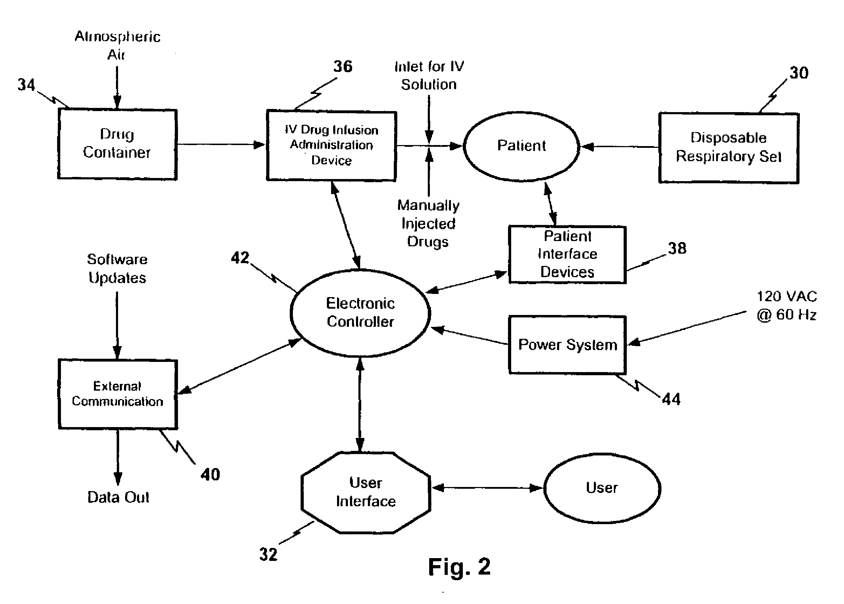

The user interface is connected to a microprocessor-based electronic controller or computer **42** (shown in **FIG. 2****)** located within housing **26.** The electronic controller or computer may be comprised of available programmable-type microprocessors and other chips, memory devices, and logic devices on various boards. The various user interface devices include a display device **33** integrated into the housing **26** of the administration device **36** which displays patient and infusion system parameters and operation status of the administration device, a printer (not shown) which prints, for example, a hard copy of patient parameters indicating the patient's physiological condition and the status of the administration device and drug flow with time stamps, and an optional remote control device (not shown) which permits a physician user to interact with the administration device from a distance. The user interface **32** includes hard and soft buttons that allow the user to override an automated drug infusion process and manually control or interrupt the drug infusion as well as purge the infusion system of air.

In a particular embodiment of the present invention, the administration device **36** is a system for providing sedation and analgesia to a patient such as the system described in U.S. Patent Application No. 09/324,759, filed June 3, 1999 and incorporated herein by reference.

**FIG. 1A** also shows a respiratory set **30** which may be attached to the administration device **36.** Preferably, the respiratory set is a single-patient or single-use disposable element that is removably attachable to the device. The administration device includes a connector port within its housing **26** in which the respiratory set may easily be attached so that it is operably coupled with the electronic controller.

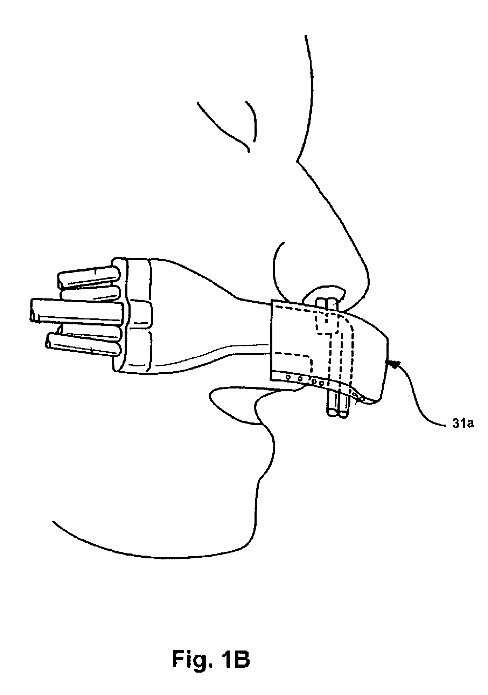

An example of the oro-nasal device **31** and respiratory set **30** that may be included in a kit of the present invention are described in U.S. Patent Application Serial No. 09/592,943, filed June 13, 2000, and U.S. Patent Application Serial No. 09/878,922, filed June 13, 2001, both of which are incorporated herein by reference. **FIG. 1B** depicts an O₂ control/respiratory monitoring device **31a,** which may be used as oro-nasal device **31** in accordance with the present invention.

**FIG. 2** is a data flow diagram showing the drug infusion management steps performed by the electronic controller **42** in a preferred embodiment of the present invention. A user interacts with a user interface **32** that is in communication with the electronic controller **42** whereby the user may input certain commands or program process sequences that are then stored in memory by the electronic controller. The electronic controller is in communication with the IV drug infusion administration device **36,** which controls flow from the drug container **34.** The electronic controller monitors and regulates the infusion rate based on input from the user and from data collected from patient interface devices. The various patient interface devices **38** can include one or more patient health monitors (not shown) that monitor a patient's physiological condition, such as a pulse oximeter, capnometer, blood pressure monitors, EEG, EKG, responsiveness query, airway pressure and others.

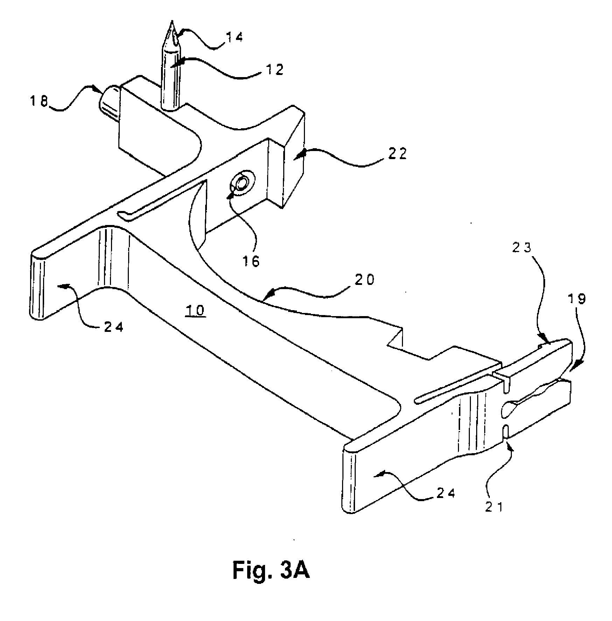

**FIG. 3A** shows a cassette **10** for the transfer of infusion liquid from a sealed drug container **34** to the patient. The cassette provides a mechanical platform for anchoring a drug container **34** to the device and assures that the drug container remains at a fixed head height with respect to the pumping mechanism **56.** The cassette also assures that the delivery conduit **27** is positioned properly with respect to the pumping mechanism. The cassette includes an extension for receiving the drug container and maintaining the container's position during the infusion process.

In a preferred embodiment, the cassette receives a single drug container for each infusion process. At the conclusion of the infusion process, the container is removed and the cassette may receive a new drug container for an extension of the first infusion process. The infusion tubing may be purged of any air and/or drug from the first infusion process. In an alternative embodiment, the cassette may receive more than one drug container at a time. The cassette may have multiple flow lumens to channel the drug flow from each of the separate drug containers into a single infusion system within the cassette or device. A mechanism may be provided to restrict the drug flow created by the pumping mechanism so that drug flows from one drug container at a time for a sequential sequence or from more than one drug container at a time according to pre-determined proportions. Pumping drug from multiple containers in tandem allows an extended infusion run without halting for a purge sequence. Pumping drug from multiple containers in concert allows separate and segregated sources of drug to be used concurrently for a single infusion run. In a further alternative embodiment, multiple containers of the same drug are provided with a single cassette such that one container can be removed while drug is flowing from another. Such an embodiment allows for an extended infusion process without halting for a purge sequence.

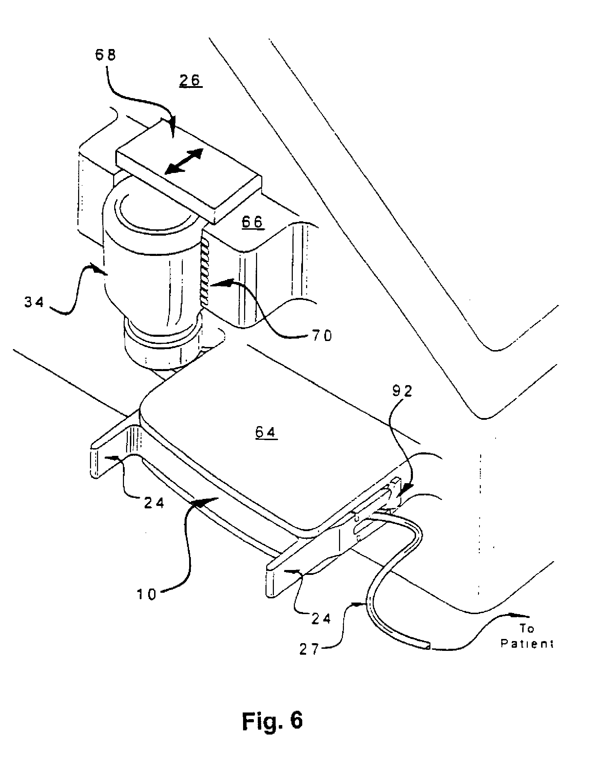

The extension may include a mechanical receptacle **66** for receiving and supporting the drug container as the infusion liquid is drawn out of the container. The receptacle may be a particular size capable of receiving a particularly sized drug container or it may be structured so as to receive containers of variable sizes. As shown in **FIG. 6****,** the receptacle **66** may be located within the housing **26** of the device **36.**

Preferably, the extension also includes an attached drug flow activation device **12** for initiating the transfer of the infusion liquid from the drug container to the device. At the start of the infusion process, the drug container is placed onto the activation device either manually or by an automated and computer controlled device for moving drug containers into position on the activation device.

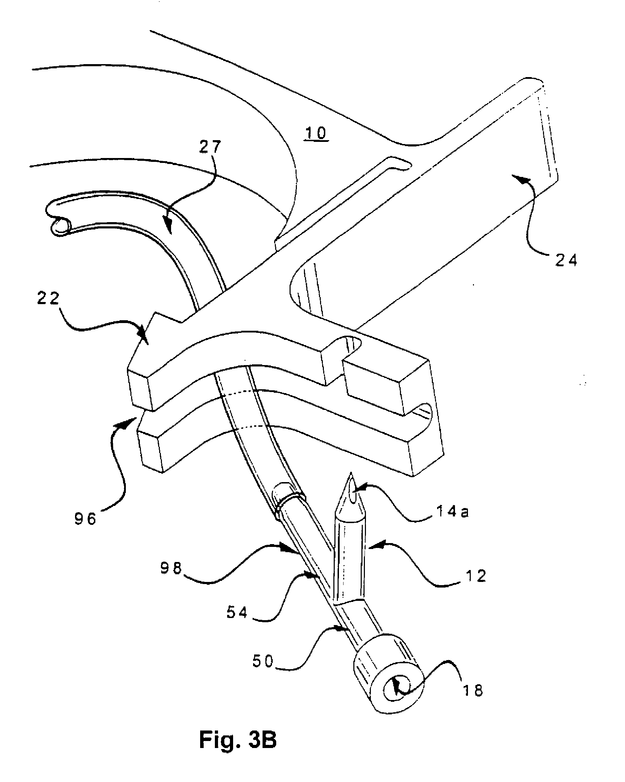

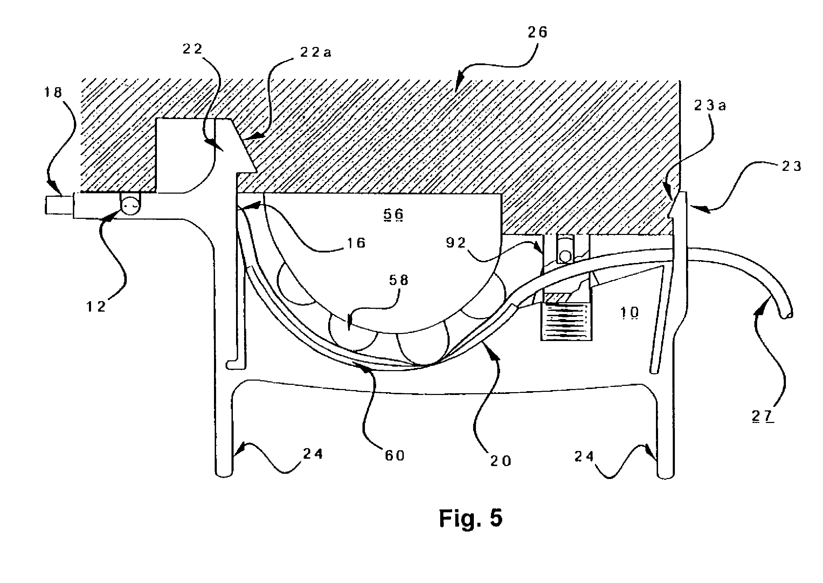

**FIG. 3A** also shows an opening and connector **16** in the cassette where the drug flow lumen **54 (****FIG. 3B****)** within the extension terminates. A pressure plate **20** is located near the opening **16.** The pressure plate is rigid enough to provide a platform against which the pump fingers **58 (****FIG. 5****)** may operate. A rigid pressure plate also allows the cassette **10** to be easily fitted onto the medical device with a one-step snap on motion. In one embodiment, the pressure plate has a concave curve that bowls away from the opening in order to accept the curved face of the pumping mechanism **56.** Alternatively, a flat pressure plate and a flat face of a pumping mechanism may also be used with the present invention.

The cassette may also include one or more extensions such as snap locks **22** and **23** which provide mechanical attachment to the housing **26** of the administration device **36** such that the cassette may be fixed in place relative to the device. In a preferred embodiment, these extensions fit into slots **22a** and **23a** on the device allowing for a snap-on single motion attachment of the cassette to the housing of the administration device. The cassette may also include extensions **24** for gripping the cassette **10** and guiding it into its designated place within the housing of the administration device. When finger grips **24** are squeezed towards each other, snap locks **22** and **23** are spread apart allowing the cassette to be placed into the slots **22a** and **23a.**

**FIG. 3B** shows how the spike **12** with an attached conduit **27** can be removed from the drug cassette **10** so that the cassette itself might be reusable. The spike set **98** fits into slot **96** when it is inserted into the drug cassette **10.** When the cassette is placed against housing **26,** housing **26** helps to keep spike set **98** securely held within slot **96 (****FIG. 5****).**

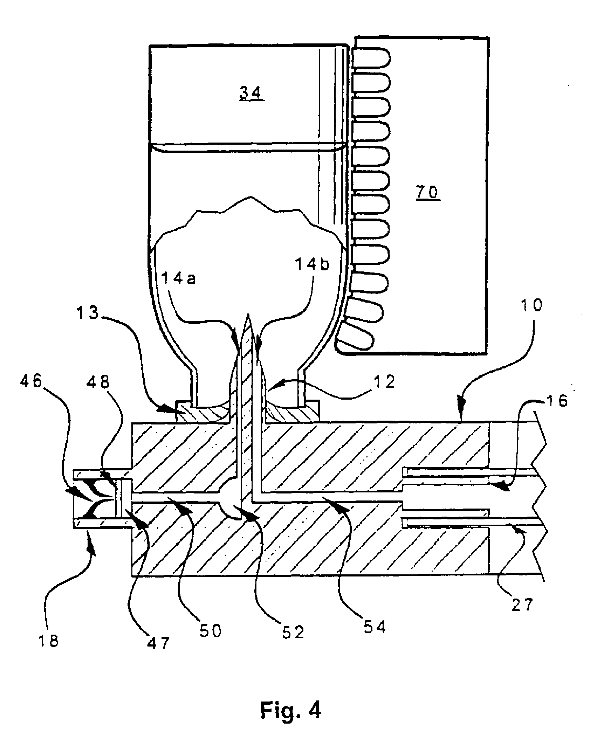

**FIG. 4** shows a preferred embodiment in which the drug flow activation device is an upright spike **12** for piercing a resealable stopper **13** of an inverted drug container **34.** The spike includes a bore **14b** that creates an air-tight opening in the container out of which the infusion liquid may flow. In an alternative embodiment, the drug container includes a pre-attached drug flow activation device and the container-activation device set may be inserted as a unit onto the extension. In a further alternative embodiment, the cassette with extension may include a pre-attached drug container with an intact, i.e., not punctured, seal which may be attached immediately prior to activation of the device. With such embodiments, the entire cassette-drug container assembly may be fixed to the administration device as a single unit, activated, and used and then may be subsequently removed from the device and disposed of as a single unit.

In a further preferred embodiment, the cassette **10** contains a drug flow lumen **54** provided between the bore **14b** and the drug flow outlet **16** in the cassette. One extremity of infusion line **27** connects to outlet **16** while the other end is attached to connector **72.** The cassette **10** may also contain an air flow lumen **50** between another bore **14a** in the spike **12** and an opening to the atmosphere through inlet **18.**

The drug infusion liquid is supplied to the device in a drug container **34.** The drug container is inert to the drug and is impermeable to atmospheric contaminants. The container is capable of protecting the drug from outside contamination prior to and during the infusion process.

Preferably, the drug container **34** is a rigid vial of invariable volume, though a flexible container such as a collapsible IV bag is also contemplated for use with the present invention. Preferably also, the drug container has at least one transparent portion to allow visual assessment of the drug's condition and volume. In a preferred embodiment, an identification tag or quality assurance module ("QAM") **35** is located on the drug container **34** and/or the cassette **10.** The identification tag **35** provides information indicating various identifiers and/or parameters of the drug, such as its name, unique serial number, concentration, and/or manufacturer identification to the user and to the electronic controller **42.**

Preferably, self-sealing stoppers **13** are used with drug containers that are to be removed from the cassette after use. Self-sealing stoppers provide air-tight piercing, prevent drug spillage, and help to prevent the drug from being compromised due to evaporation or contamination.

Preferably also, the drug container includes a built-in gripping device such as a molded tab (not shown) by which a user can hold and transport the container without contaminating its surface.

**FIG. 4** also shows a further preferred embodiment in which the extension to cassette **10** includes a one-way valve **46** through which atmospheric air is introduced into a rigid drug container like a vial in order to prevent excessive vacuum (that would interfere with drug infusion) from developing above the liquid drug's meniscus as the drug flows out of the container. An air flow lumen **50** is provided between one-way valve **46** and bore **14a** in spike **12.** Because in the embodiment depicted in **FIG. 4** drug can flow by gravity along air flow channel **50** to the atmosphere, certain embodiments are contemplated to prevent drug from leaking out of the air flow lumen while still allowing air to bleed inside the drug container to prevent formation of an excessive vacuum. In one of these embodiments, the mechanism to prevent drug spillage from bore **14a** is a one way valve **46.** The one-way valve **46** only allows atmospheric air into the air flow lumen **50** and does not allow any drug which has leaked through the bore **14a** to escape the cassette. In a further drug leakage prevention embodiment, a hydrophobic filter **47** is provided with the air flow lumen **50** in the extension. The hydrophobic filter prevents any drug which has leaked into the air flow lumen **50** of the spike from flowing out of the air inlet **18** of the cassette. In a further drug leakage prevention embodiment, bore **14b** is a wide bore and bore **14a** is a narrow bore. The narrow bore **14a** is in communication with the air flow lumen **50** in the extension while the wide bore **14b** is in communication with the drug flow lumen **54** of the extension. The difference in capillary action caused by the different bore sizes causes the liquid drug in the drug container to tend to flow through the wide bore **14b** and into the drug lumen **54** only. Capillary action hinders the flow of drug into the narrower air flow lumen. In a further drug leakage prevention embodiment, the air flow lumen **50** contains a half-moon-shaped well **52** so as to restrict the flow of any drug that does leak into the air flow lumen **50** from making it to the air inlet **18.**

Preferably, an air filter **48** is provided with the air inlet **18** to prevent particulates and contaminants in the atmospheric air from entering the air flow lumen **50** inside the extension and the drug container **34.** The air filter **48** may be capable of screening out microbial matter including bacterial and viral particles.

**FIG. 5** shows a drug delivery conduit **27** that is provided with the cassette at opening and connector **16.** Conduit **27** is inserted over the male port in opening **16** to create an air-tight connection with the flow channel created by the drug flow lumen **54.** The conduit **27** is positioned along the pressure plate such that the pumping mechanism **56** may act on it to move the infusion liquid through the conduit, away from the drug container, and to the patient. Preferably, the conduit, which may be tubing, is fixed in position along the pressure plate. Because of the concavity of a certain embodiment of the pressure plate, the conduit **27** will tend to straighten out and not remain in contact with a concave pressure plate. A means to hold the conduit abutted against the pressure plate should not interfere with the action of the peristaltic pump fingers **58.** Several embodiments are contemplated for fixing the conduit to the pressure plate. For example, the conduit may be ultrasonically welded or glued to the plate or it may be fitted within foam strip guides **60,** which are themselves fixed to the pressure plate. The foam strip guides by virtue of being compressible and collapsible do not interfere with the accuracy of the pump or the operation of the pump fingers **58.** Alternatively, pieces of plastic tubing similar to conduit **27** could be placed on the pressure plate **20** above and below conduit **27** such that they hold conduit **27** securely against pressure plate **20** and collapse when squeezed by the pump fingers **58.**

The delivery conduit **27** is positioned against the cassette such that when the cassette is fitted into the housing **26** of the device, conduit **27** is properly positioned with respect to the pumping mechanism **56.** In a preferred embodiment, at least a portion of the delivery conduit **27** is transparent so that the user can observe the drug flow through the conduit and visually check for entrained air bubbles or particulates in the drug.

**FIG. 5** also shows the pumping mechanism **56** which aligns with the pressure plate **20** of the cassette when the cassette is fitted into the housing of the administration device. The pumping device may be a peristaltic pump with at least three, and preferably at least four, movable fingers **58** which act upon the delivery conduit **27** and against the pressure plate **20** so as to create a pressure gradient within the delivery conduit. The pressure gradient causes the infusion liquid to flow from the drug container into the drug flow lumen within the spike, then into the drug flow lumen within the cassette extension, then into the delivery conduit, and then through the manifold connector **72** and into the IV cannula **84** inserted in a vein of the patient. Because the fingers are external to the delivery conduit and the entire infusion system tubing, the pumping mechanism is able to operate even if air is in the active pumping section of the delivery conduit. The pumping mechanism may be controlled manually or by the electronic controller and may be set at a given flow rate or at a specified gradient, rate of change over time or time profile of drug flow rates.

**FIG. 5** also shows spring-loaded clamp **92,** which acts as a free flow prevention device to halt the unchecked or free flow of drug to the patient by gravity when the cassette is removed from contact with the pumping mechanism. Clamp **92** pinches a portion of the conduit **27** closed when triggered.

**FIG. 6** also shows an array **70** of photo-emitter cells and photo-detector cells, which is an element used in an alternative redundant volume tracking method. Such an array may be provided with the cassette or as part of the administration device **36.** Each photo-emitter cell emits a pulse of light directed at the drug container. The difference in reflection of the emitted light depending on whether it impinges on air or drug, especially milky drugs like propofol, is used to track the meniscus. Emitted light which reflects back from the liquid inside of the container is detected by a photo-detector cell. The detector cells are capable of receiving reflected light from the drug and are arranged in a pattern, such as a column, whereby if a particular detector cell receives a certain amount of reflected light, then it is below the meniscus of the drug and whereby if the particular detector cell receives a different amount of reflected light, then it is above the meniscus of the drug. Each cell of the array is in communication with the electronic controller and the controller determines where the meniscus is within the drug container by identifying the region where there is a sharp transition in the reflected light. Meniscus tracking allows the controller to independently calculate how much drug remains in the drug container based on the initial volume of the liquid drug in the container. The initial volume of the liquid drug in the container may be encoded on a QAM unit located on the container. A mechanism is provided to read the information on the QAM and transmit the encoded value of the initial volume to the electronic controller. The photo emitter/detector pairs may be staggered in two separate columns to provide more vertical resolution.

**FIGS. 3** and **6** also show a further embodiment of a free flow prevention device. Snap lock **23** of cassette **10** contains a slit **19** through which the drug delivery conduit **27** is placed. Cut-outs **21** are provided at each side of the slit to allow the slit to be forced wide apart such that when the cassette is placed into proper position on to the device housing **26** a spreader piece **92** located on the housing spreads the fingers of snap lock **23** allowing the unrestricted flow of liquid through conduit **27.**

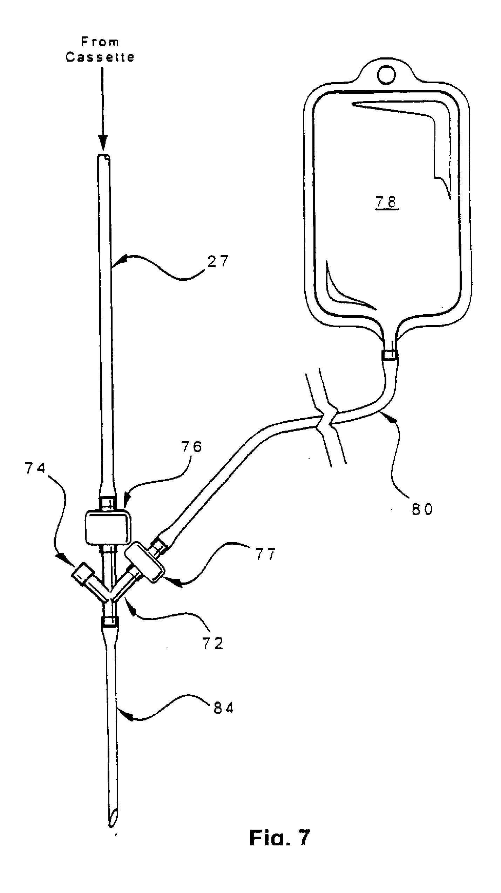

**FIG. 7** shows an anti-reflux valve **77** on connector **72** connecting delivery conduit **27** with the tubing **80** from the IV solution container **78** and the IV catheter **84.** The anti-reflux valve **77** prevents the retrograde flow of drug from tubing **27** into the IV solution tubing **80.**

A check valve **76** that is part of connector **72** prevents back flow of intravenous fluid up the propofol line **27.** Check valve **76** can also operate as an automated free flow prevention device by deliberately increasing its cracking or opening pressure such that it is higher than the highest hydrostatic pressure generated by a spiked and full drug vial with conduit **27** fully extended to its highest possible elevation. The design thus requires the pumping mechanism **56** to generate more pressure than the opening pressure of valve **76** for drug to flow to the patient. If the pump mechanism is not in contact with conduit **27** and pressure plate **20,** when the cassette **10** is removed from housing **26** for example, drug flow will stop because the highest hydrostatic head that can be generated will be lower than the cracking pressure of valve **76.**

In an alternative embodiment, the connector **72** may also include a resealable injector port **74** capable of accepting a syringe tip and/or needle and allowing the direct injection of drugs therefrom. An IV catheter **84** may be inserted into the patient's blood vein. Preferably, the IV catheter is a single-patient or single-use disposable element that is removably attachable to the device.

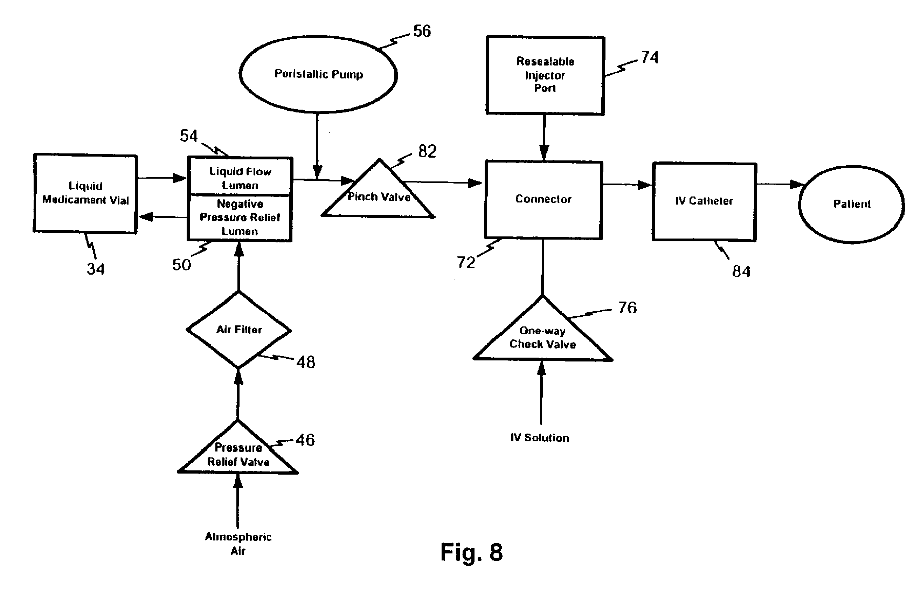

**FIG. 8** shows a block diagram of an embodiment of the present invention and depicts the infusion liquid and atmospheric air flow pathways through the elements described above. Pinch valve **82** is open when the cassette is snapped onto housing **26.** As soon as cassette **10** is snapped off, the spring in pinch valve **82** closes off IV line **27.** The purpose of pinch valve **82** is to prevent free flow of drugs by gravity to the patient, when flow through conduit **27** is no longer being controlled by pumping mechanism **56** because conduit **27** is no longer in contact with it.

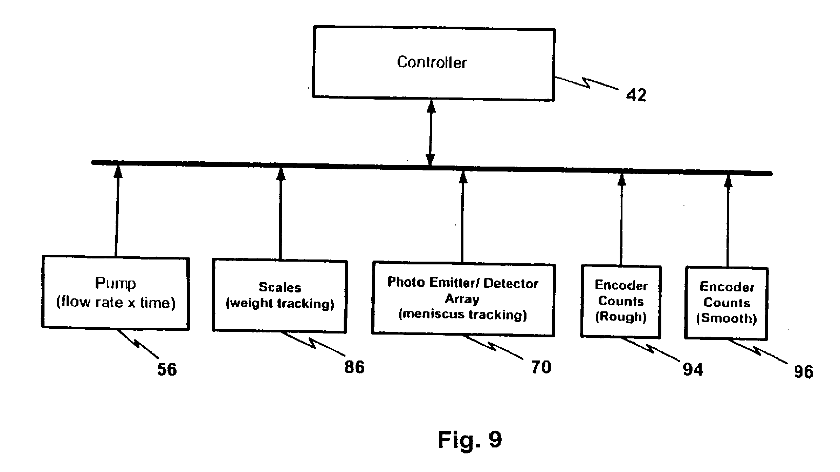

**FIG. 9** shows various mechanisms for tracking the volume of infusion liquid pumped out of the drug vial during the infusion process. Methods for volume tracking provide redundancy to the volume calculated by the electronic controller from the flow rate of the pumping mechanism and the duration of the infusion so that the accuracy of the pumping mechanism's flow rate may be verified and compensated for. This redundancy ensures a dependable and accurate flow rate of drug into the patient.

One such mechanism for redundant volume tracking utilizes scales which measure the weight of the drug container as it is in contact with the drug flow activation device. The scales may be provided with the cassette or as part of the administration device. The scales are in communication with the electronic controller which receives either continuous or periodic data on the weight of the drug container and its remaining contents. As drug flows out of the container, the weight decreases and the electronic controller calculates the corresponding decrease in drug volume from a preprogrammed set of drug density data.

Another redundant volume tracking mechanism is the photo emitter/detector array for meniscus tracking described above. The array **70** of photo emitter/detector cells will track the meniscus of the drug, but for the controller **42** to translate a change in meniscus position to a change in volume infused, the cross-sectional area of the vial must be known. The internal cross-sectional area of vial **34** can be stored in a QAM attached to the vial **34.**

Another redundant volume tracking means is provided by tracking internal encoder counts of the pumping mechanism. Because most pumps use a motor to drive the pumping mechanism, there should be a set volume of drug delivered with each revolution of the pump's motor. If an encoder mechanism, such as a set of optical emitter/detector cells capable of detecting the passage of slots in the pump's cam, is provided with the pump, each revolution of the pump's motor can be detected. The electronic controller can multiply the number of revolutions per minute of the pump's motor by the volume of drug delivered per revolution to get the infusion rate in volume per minute. The controller can then integrate rate over time to calculate the total volume infused over time.

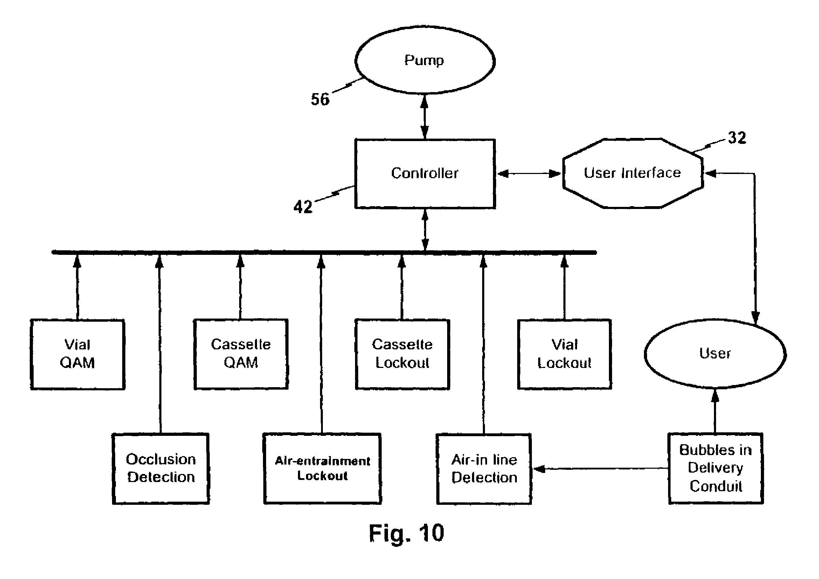

**FIG. 10** shows various optional methods for alerting the electronic controller of reason to shut off the pumping mechanism. These methods help to prevent air from being pumped into a patient's blood circulation and help to prevent an incorrect (e.g., expired, previously used, or unrecognized) drug or an incorrect dose from being administered to a patient.

In one of these methods, the user manually signals for a pump shut down if bubbles are observed in the delivery conduit. The user interacts with a user interface which is in communication with the electronic controller. An air-in-line detector may also be provided within the device to sense air bubbles within the infusion liquid pumped into the device. The air-in-line detector is in communication with the electronic controller. The electronic controller may be programmed to send a signal to the pumping mechanism to terminate the flow rate upon notice of a signal from the air-in-line detector. The conduit or PVC tubing may then be purged of the trapped air.

In another of these methods, an occlusion detector is provided with the device to sense via the associated pressure buildup when a kink or obstruction to flow is present in the infusion liquid delivery line. The occlusion detector is in communication with the electronic controller and sends a signal to the controller when such an obstruction is detected. The controller may be programmed to send a signal to the pumping mechanism to terminate the flow rate upon notice of a signal from the occlusion detector.

In yet another of these methods, an air-entrainment lockout mechanism is provided with the cassette or with the device. An air-entrainment lockout mechanism is triggered by the removal of a drug container from the cassette while the pumping mechanism is running. Once triggered, the air-entrainment lockout mechanism halts the flow of drug within the cassette.

An example of an air-entrainment lockout mechanism is a micro-switch located on or near the drug flow activation device. When the drug container is removed from the activation device it triggers the micro-switch to send a signal to the electronic controller. The micro-switch may be a spring-loaded button that is depressed as long as the drug container is on the activation device and is released when the container is removed, it may be a spring-loaded button positioned in such a location as to be depressed by the surface of the drug container as the container is removed, or it may be an electronic sensor such as an optical or electromagnetic sensor that registers when the drug container is removed.

In a preferred embodiment, a drug container removal lockout mechanism **68** (shown in **FIG. 6****)** is provided with the housing to prevent the removal of the container **34** while the pumping mechanism is running. When in a locked position, the mechanism **68** slides out of housing **26** and mechanically prevents removal of the drug container from the cassette. The mechanism **68** may be in communication with the electronic controller, which will only signal the pumping mechanism to run when the lockout mechanism is in a locked position. When mechanism **68** is in an unlocked position and retracted into housing **26,** the drug container may be physically removed from the cassette and the electronic controller will signal the pumping mechanism to halt the drug flow. Once a new drug container is inserted on the cassette and the lockout mechanism is returned to a locked position, the electronic controller will again signal the pumping mechanism to run. If the electrical power or software to the controller **42** fails, the mechanism **68** can be manually pushed back into housing **26** to allow removal of the vial **34.** This feature of the present invention removes the need for a purging sequence each time a drug container is removed and replaced by another container containing a drug with the same identity and concentration of the drug of the first container.

In another of the optional methods for alerting the electronic controller to shut off the pumping mechanism, various quality assurance modules attached to the cassettes and vials are contemplated which store information to be communicated to the electronic controller. If a parameter recorded on a QAM is out of a preprogrammed range stored in memory by the electronic controller, then the controller may send a signal to the pumping mechanism to terminate the flow rate.

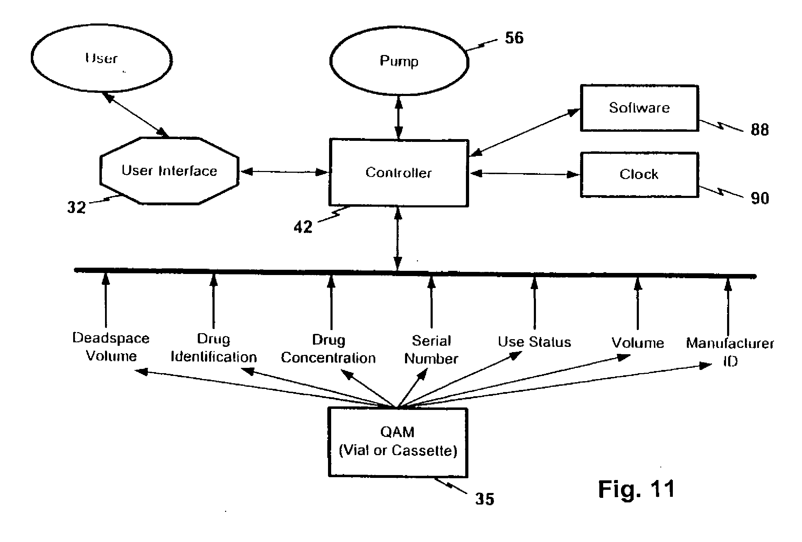

**FIG. 11** is a block diagram of certain parameters that the drug container QAM and cassette QAM may store. Tags on the drug container or cassette may store such parameters as the identity, concentration, initial volume of a drug, serial number, and manufacturer identification in the form of a barcode or RFID tag for example.

The electronic controller receives the parameter data from the QAMs and processes it to determine the initial conditions of the infusion setup. The controller may use drug identity data encoded on a tag to authenticate product source and ensure that the particular drug to be infused is the drug intended for the current patient.

The electronic controller may also use the drug identity information encoded on the drug container or cassette tags to determine when cross-contamination may occur. The controller may store in memory the identity and concentration of a first drug in use and the identity and concentration of a second drug to be used with the same cassette and device. If the stored identity or concentration of the second drug is different from the first drug, the electronic controller will automatically initiate a purging sequence to clear any residual drug from the first infusion sequence from the system.

In a preferred embodiment, the electronic controller uses data from the QAMs to coordinate an automatic purging sequence. A QAM on the cassette may store the deadspace volume of the drug flow lumen and delivery conduit of the cassette. The electronic controller records these deadspace volumes from the QAMs and signals the pumping mechanism to cause a volume of drug in excess of the sum of the deadspace volume of the cassette and device tubing to flow through the infusion set to clear any air remaining in the lines. An automatic purging sequence allows for the precise control of volume of drug pumped through the system during a purge sequence so that just enough volume of drug is pumped to assure that the infusion set is free of trapped air. Such a purging sequence performed manually may result in a greater than necessary volume of drug being pumped out of the infusion system resulting in wasted drug.

Preferably, the electronic controller references a clock to establish the start time and duration of each infusion run. The controller may also use the clock to determine when preprogrammed events such as pump flow rate or drug container changes should occur. The controller may also use the clock and the infusion rate over a given time period to determine how much drug is left in the container so as to shut off the pump when the volume of drug remaining in the container is low and alert the user.

**FIG**. **12** shows an alternative embodiment in which the drug flow activation device **12** allows transfer of infusion liquid from an upright drug container. An elevator **94** is used to raise an upright drug container **34** into communication with the activation device. Preferably in this embodiment, an inverted spike is used as the activation device. The electronic controller may be programmed to automatically operate the elevator or the elevator may be operated manually.

The present invention also provides specialized kits of components or supplies for use with the administration device **36.** These kits may comprise disposable and/or re-usable components, supplies that are intended or designed solely for use with the administration device **36,** commonly-used medical supplies, supplies needed for drug administration, and medical supplies required for a specific procedure (e.g., endoscopy) to be performed as accompanied by drug administration. The kits may comprise wholly re-usable items, a mix of re-usable and disposable items, or only disposable items.

The kits of the present invention promote the efficiency and safety of delivering drugs using device **36.** The user does not have to individually collect the separate supply items needed to deliver sedation and analgesia, whereby optimizing time and motion. In embodiments where the kit also includes the supplies needed for a particular procedure, there is no need for a user to collect the supplies for the procedure separately, sometimes from a separate location. This also optimizes time and motion.

The packaging for a kit in accordance with the present invention may contain recesses for individual components and supplies, and may be transparent so that the user can see and examine the contents of the kit without having to first open the package. The packaging may be made of inexpensive material such as, for example, plastic, that can be sterilized or irradiated as required to ensure safety. The package may be closed with a snap-lock system that is tight and secure when closed, but still allows one-handed opening by a user with a gloved hand. The components of a kit may be laid out in an ergonomic manner that facilitates the user locating, retrieving and/or safely using the components. For example, the components may be placed within a kit such that their orientation is appropriate for installation with administration device 36 by a right-handed user with a minimum of movement and manipulation of the component or supply. Similar kits designed and optimized for left-handed users are also contemplated. Similarly, the relative placement of the components in the kit may be based according to their logical, expected sequence of use. For example, sharp or pointed supplies like scalpels may be oriented so that the risk of injury to the user or to bystanders is minimized when the item is picked up and retrieved. A kit package may have recesses that house each component and may be constructed so as to lay flat and stable with a minimal footprint, when opened. The recesses may each be labeled with the name of their respective component so as to assist in their identification by a novice user. The package itself may contain identification and use status indicia as well as markers to confirm that the package has undergone a cleaning or sterilization process such as, for example, ethylene dioxide or gamma ray. The package may also be designed to be as small as possible so that it occupies a minimal amount of work area and/or shelf space during storage. In some instances, the sedation and analgesia kit package may also have double sided tape or other adhesive or anchoring devices, such as hook-and-loop fasteners (Velcro) or magnets, on the bottom to allow the kit to be temporarily affixed to a work surface so that the kit package does not move around as it is being used, especially during one-handed use.

The components of or supplies used with the administration device **36** that are included in a kit may include identification indicia, such as a tag or QAM **35,** for quality assurance, identification, and safety purposes, where supplies may be designed, for example, to prevent cross-contamination and use past an expiration data. The package housing the kit may itself also incorporate identification and use status indicia so that its use status and history as well as other relevant data may be available to the sedation and analgesia delivery system. Examples of such identification indicia and particular means by which their information is written and read are disclosed by U.S. Patent Application Serial Nos. 10/151,255 and 101252,818, filed May 21, 2002, and September 24, 2002, respectively, and incorporated herein by reference.

Several of the components or supplies described above for use with administration device **36** are contemplated as being included in a kit according to the present invention. Examples of such components and supplies that may be provided in a kit include but are not limited to the cassette **10,** air filter **48,** air flow lumen **50,** double lumen spike **12** or spike set **98,** free flow prevention devices such as spring-loaded clamp **92,** snap lock **23,** and pinch valve **82,** drug delivery conduit **27,** pumping mechanism **56,** anti-reflux valve **77,** connector **72,** IV tubing **80,** check valve **76,** IV catheter **84,** IV solution container **78,** and drug vial **34.** Further components that may be included in a kit that are for systems ancillary to administration device **36** which may be used during the procedure accompanied by drug administration include but are not limited to ECG pads, respiratory set **30** and oro-nasal device **31,** Bispectral index (BIS) monitoring strips, water traps, and an cover for earpiece **37.** An example of earpiece **37** is described in U.S. Patent Application Serial No. 10/329,763, filed December 27, 2002.

The kit of the present invention may include supplies for use with a procedure that is performed as accompanied by drug delivery from device **36.** Examples of such supplies include but are not limited to a trocar, stapler, and biopsy forceps for an endoscopy; a bite block, endoscope, local anesthetic sprayer, local anesthetic, and biopsy forceps for an EGD; a colonoscope, gauze for holding the colonoscope, local anesthetic gel, and biopsy forceps for a colonoscopy; and a laparoscope, trocars, local anesthetic, needle and syringe for local anesthetic, prep solution (e.g., betadine), prep applicator, sterile field drape, and a scalpel for making initial hole through skin for a laparoscopy or a arthroscopy. The kit may also include standard medical supplies for use with a variety of procedures that may be accompanied by drug delivery from device **36.** Examples of standard medical supplies that may be included in a kit include but are not limited to alcohol preps, betadine, stericides, stericide applicators, lubricants, medical tape, suture, needles, scalpels, syringes, drugs, and special adapters and connectors.

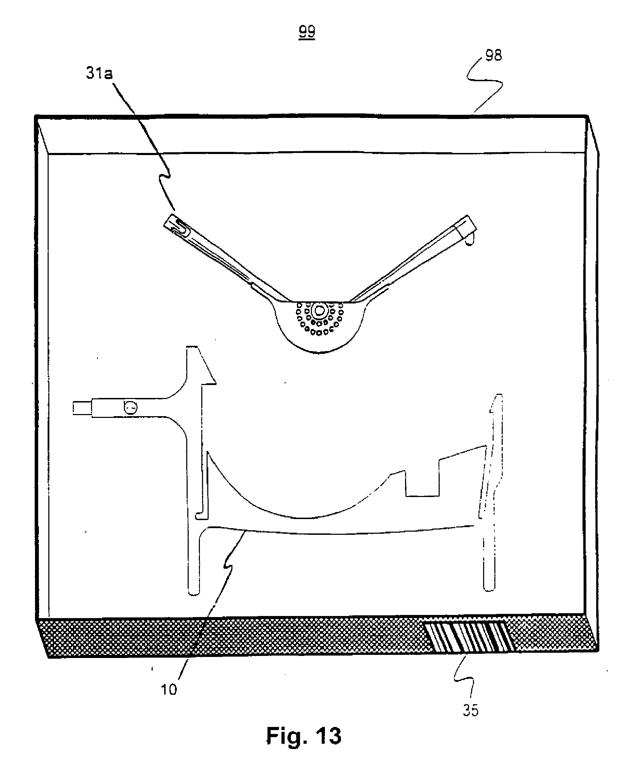

Purely by way of example, **FIG. 13** depicts a kit **99** according to the present invention wherein a cassette **10** and an 0₂ control/respiratory monitoring device **31a** are provided in the same kit and wherein the kit **99** features a QAM **35** on its packaging **98.**

While exemplary embodiments of the invention have been shown and described herein, it will be obvious to those skilled in the art that such embodiments are provided by way of example only. Numerous insubstantial variations, changes, and substitutions will now be apparent to those skilled in the art without departing from the scope of the invention disclosed herein by the Applicants.

## Claims

1. A system for enabling safe sedation and/or analgesia to a patient by an non-anesthetist during a medical procedure, said system comprising:
an electronically controllable drug delivery device (36) capable of controlling flow of a sedative and/or analgesic to a patient during said procedure,
a patient health monitor (38) that collects and provides data concerning a patient's physiological condition to a controller,
an electronic controller (42) adapted to receive identification and/or use indicia of disposable components for use with said system and to regulate an infusion rate of said drug based upon input from a user and upon said data from said patient health monitor; and
a pre-packaged kit of disposable components for use with said system, said components being selected from the group of: a drug cassette, a drug, infusion tubing, a respiratory set, EKG pads, skin electrodes, and IV catheters; wherein one or more of said components carries a quality control and/or source marker providing said identification and/or use indicia to insure said non-anesthetist that the appropriate disposable components are to be used with said system.

2. A system as recited in claim 1, wherein said quality control and/or source marker comprises an RFID.

3. A system as recited in claim 1, wherein said quality control and/or source marker comprises a barcode.

4. A system as recited in claim 1, wherein said quality control and/or source marker comprises a tag associated with said component.

5. A system as recited in claim 1, wherein said quality control and/or source marker includes identification of source, quality, and/or safety.

6. A system as recited in claim 1, wherein said drug has an initial volume, intermediate volume, and final volume during a medical and/or surgical procedure, and said volume is measured by the controller and a method selected from the group consisting of weight of drug, meniscus of drug surface, and rotation of pump mechanism to measure the volume in redundancy.

7. A system as recited in claim 1, wherein said delivery device can be manually controlled.

8. A system as recited in claim 1, wherein the electronic controller receives said identification and single use status indicia of drug delivery components and determines if cross-contamination may occur.

9. A system as recited in claim 8, wherein the electronic controller automatically initiates a purging sequence if cross-contamination may occur.

10. A system as recited in claim 1, wherein said kit further includes other disposable components for use with said system.

11. A system as recited in claim 10, wherein said other components include a free flow prevention device to halt the unchecked or free flow of drug through a drug delivery conduit.

12. A system as recited in claim 11, wherein said free flow prevention device is at least one of a spring loaded clamp, a snap lock, and a pinch valve.

13. A system as recited in claim 2, wherein said RFID contains identification and use status indicia.

14. A system as recited in claim 1, wherein the packaging of said kit includes means for opening and removal of said components with a single hand.

15. A system as recited in claim 1, wherein the packaging of said kit contains anchoring means to allow said kit to be temporarily affixed to a work surface such that said kit is immobile and said components can be removed without moving said packaging.

16. A system as recited in claim 1, wherein the packaging of said kit is sufficiently translucent to allow visual identification of said components through said packaging.
